# EUROPÄISCHE PATENTSCHRIFT

(11) **EP 3 119 377 B1**
(45) Veröffentlichungstag und Bekanntmachung des Hinweises auf die Patenterteilung: **29.12.2021**
(21) Anmeldenummer: 15710542.0
(22) Anmeldetag: 19.03.2015
(51) Int. Cl.: A61K 6/17, A61K 6/20, A61K 6/61, A61K 6/62, A61K 6/64, A61K 6/887, A61K 6/889

(54) **PHOTOPOLYMERISIERBARE UND DUALHÄRTENDE DENTALWERKSTOFFE AUF DER BASIS VON THIOHARNSTOFFDERIVATEN**
PHOTOPOLYMERISABLE AND DUAL HARDENING DENTAL MATERIAL BASED ON THIOUREA DERIVATIVES AND BISACYLDIALKYLGERMANIUM-COMPOUNDS
MATÉRIAUX DENTAIRES PHOTOPOLYMÉRISABLES À DOUBLE POLYMÉRISATION À BASE DE DÉRIVÉS DE THIO-URÉE ET DE BISACYLDIALKYLGERMANIUM

(30) Priorität: 20.03.2014 EP 14160824
(43) Veröffentlichungstag der Anmeldung: 25.01.2017
(73) Patentinhaber: Ivoclar Vivadent AG, 9494 Schaan (LI)
(72) Erfinder: MOSZNER, Norbert, FL-9495 Triesen (LI); BURTSCHER, Peter, A-6830 Rankweil (AT); GIANASMIDIS, Alexandros, CH-9436 Balgach (CH)
(74) Vertreter: Uexküll & Stolberg
(86) Internationale Anmeldenummer: PCT/EP2015/055858
(87) Internationale Veröffentlichungsnummer: WO 2015/140276

(56) Entgegenhaltungen:
- US-A- 3 991 008
- US-A- 4 612 384
- US-A1- 2008 277 814
- US-A1- 2010 068 679
- None

## Beschreibung

Die vorliegende Erfindung betrifft photopolymerisierbare und dualhärtende dentale Werkstoffe, die sich insbesondere zur Verwendung als dentales Füllungsmaterial oder Befestigungszement eignen.

Dentale Komposite enthalten gewöhnlich eine polymerisierbare organische Matrix und einen oder mehrere Füllstoffe. Als polymerisierbare organische Matrix wird in den meisten Fällen eine Mischung von Monomeren, Initiator-Komponenten, Stabilisatoren und Pigmenten verwendet, wobei als Monomere oft Mischungen von Dimethacrylaten eingesetzt werden. Die Aushärtung derartiger Materialien kann durch thermische, redoxinitiierte oder lichtinduzierte radikalische Polymerisation erfolgen. In zunehmendem Maße werden auch acide Monomere zur Herstellung von Dentalmaterialien verwendet. Diese verleihen den Materialien selbstätzende Eigenschaften und verbessern ihre Haftung an der natürlichen Zahnsubstanz.

Zur Härtung der Materialien werden im Falle von indirekten Füllungsmaterialien überwiegend thermische Initiatoren, wie z.B. Dibenzoylperoxid (DBPO), oder Derivate der Barbitursäure eingesetzt, wie z.B. Trimethylbarbitursäure. Für bei Härtung bei Raumtemperatur werden die Peroxide mit Aminen wie N,N-Dimethyl-sym.-xylidin oder N,N-Dimethyl-p-toluidin und die Barbitursäurederivate mit Perverbindungen wie z.B. Kaliumperoxosulfat oder Perestern kombiniert. Sowohl Peroxide als auch Barbitursäurederivate müssen aufgrund ihrer begrenzten thermischen Stabilität im Kühlschrank gelagert werden.

Als Photoinitiatoren für lichthärtende Materialien haben sich α-Diketone bewährt, wie z.B. Campherchinon (1,7,7-Trimethylbicyclo[2.2.1]heptan-2,3-dion) (CQ) und 9,10-Phenanthrenchinon. Photoinitiatoren werden in der Regel zusammen mit Aminen wie 4-(N,N-Dimethylamino)-benzoesäureethylester als Reduktionsmittel eingesetzt. Dualhärtenden Dentalmaterialien enthalten eine Mischung von einem Photoinitiator und einem Redox-Initiatorsystem.

Die Verwendung von Aminen als Initiatorkomponente hat eine Reihe von Nachteilen. So kann die Oxidation der Aminbeschleuniger zu Verfärbungen der Werkstoffe führen. Außerdem reagieren Amine leicht mit aciden Stoffen und sind daher in Anwesenheit von sauren Monomeren nicht stabil.

Eine sehr gute Säurestabilität zeigen Redox-Initiator-Systeme auf der Basis von Thioharnstoff-Derivaten.

Die US 3,991,008 und die DE 26 35 595 Al offenbaren polymerisierbare Zahnfüllmassen auf der Basis von Methacrylatmonomeren, die als Initiator ein Hydroperoxid-Oxidationsmittel in Kombination mit einem substituierten Thioharnstoff als Reduktionsmittel enthalten. Ein bevorzugtes Oxidationsmittel ist Cumolhydroperoxid, ein bevorzugtes Thioharnstoffderivat Acetylthioharnstoff. Die Zusammensetzungen sollen sich durch verbesserte Farbbeständigkeit und Lagerfähigkeit auszeichnen und keine Lagerung im Eisschrank erfordern. Zudem sollen sie eine hervorragende Härtegeschwindigkeit aufweisen.

Aus der EP 1 479 364 Al sind selbstätzende Dentalmaterialien bekannt, die neben einem substituierten Thioharnstoff und einem Hydroperoxid mindestens eine acide Komponente enthalten. Die Materialien sollen eine gute Lagerstabilität aufweisen und werden vorzugsweise in zweikomponentiger Form bereitgestellt, wobei die eine Komponente den substituierten Thioharnstoff und die zweite Komponente das Hydroperoxid enthält. Zur Herstellung dualhärtender Materialien kann ein Photoinitiator zugesetzt werden.

Die US 2003/0134933 offenbart zweikomponentige, selbsthärtende Wurzelkanalfüllungsmaterialien, die sich durch eine hohe Stabilität bei Temperaturen bis zu 60°C auszeichnen sollen. Die Materialien enthalten als Initiator ein Hydroperoxid in Kombination mit einem Thioharnstoffderivat. Zur Beschleunigung der Redoxreaktion bei Raumtemperatur kann die thioharnstoffhaltige Komponente zusätzlich eine acide Verbindung enthalten. Um eine schnelle Härtung der Materialien im oberen Teil des Wurzelkanals zu ermöglichen, können Photoinitiatoren eingesetzt werden.

Die WO 03/057792 A2 beschreibt die Verwendung polymerisierbarer Harnstoff- und Thioharnstoffderivate als Reduktionsmittel in Redox-Initiatorsystemen. Die Harnstoff- bzw. Thioharnstoffverbindungen sollen in Kombination mit einem zweiten Reduktionsmittel wie zum Beispiel Ascorbinsäure oder einem nicht polymerisierbaren Thioharnstoffderivat eine hohe Farbstabilität sowie gute Lagerstabilität und Härtungseigenschaften erheben. Das Reduktionsmittel wird mit einem Oxidationsmittel und gegebenenfalls einem Photoinitiator kombiniert.

Gemäß EP 1 754 465 Al soll sich die Initiatorwirkung von Thioharnstoff/Hydroperoxid-Initiatorsystemen durch die Zugabe katalytischer Mengen an Kupferverbindungen verbessern lassen. Als geeignete Kupferverbindungen werden Kupfersalze und -komplexe genannt, wie zum Beispiel Kupferbenzoat, Kupferdi-(methacrylat), Kupferacetylacetonat und Kupfernaphthenat.

Die EP 1 693 046 Al offenbart dentale Zusammensetzungen, die als Initiatorsystem ein 2-Pyridylthioharnstoffderivat und ein Hydroperoxid enthalten. Die Zusammensetzungen sollen sich durch eine hohe Säuretoleranz auszeichnen und sich zur Herstellung acider dentaler Primer und Adhäsive eignen. Sie können zusätzlich übliche Photoinitiatorsysteme enthalten.

Die WO 2008/134024 A2 schlägt als Initiatorsystem für acide selbstätzende Dentalzemente die Verwendung von Cumolhydroperoxid in Kombination mit Benzoylthioharnstoff vor. Die Wirksamkeit dieses Initiatorsystems soll durch acide Monomere nicht beeinträchtigt sondern vielmehr verstärkt werden. Die Zusammensetzungen können zusätzlich einen Photoinitiator enthalten.

Die EP 2 233 544 A1 offenbart zweikomponentige, polymerisierbare Dentalwerkstoffe, die ein Hydroperoxid, ein Thioharnstoffderivat als Reduktionsmittel und eine Vanadiumverbindung als Beschleuniger enthalten. Außerdem enthalten die Werkstoffe ein Polymer einer α,β-ungesättigten Mono- oder Dicarbonsäure. Sie sollen eine gute Härtbarkeit und Stabilität aufweisen.

Ausserdem werden monomolekulare Photoinitiatoren eingesetzt, wie beispielsweise die kommerziell erhältlichen Verbindungen 2,4,6-Trimethylbenzoyldiphenylphosphinoxid und Bis(2,4,6-trimethylbenzoyl)phenylphosphinoxid. Diese Photoinitiatoren bilden in einer monomolekularen Elementarreaktion Radikale, d.h. durch Bindungsspaltung des Photoinitiators.

Neuerdings werden auch Acylgermane als Photoinitiatoren für Dentalwerkstoffe eingesetzt. Die EP 1 905 413 Al offenbart Acylgermane, die sich mit sichtbarem Licht im Wellenlängenbereich von 200 bis 750 nm aktivieren lassen. Diese zeichnen sich durch eine hohe Reaktivität aus und sind daher bereits in geringen Konzentrationen aktiv. Sie sind in Anwesenheit acider Monomere stabil und führen sie nicht wie herkömmliche Photoinitiatoren für den sichtbaren Wellenlängenbereich zu Verfärbungen der Materialien. Acylgermane können zur Herstellung dualhärtender Materialien mit herkömmlichen Redox-Systemen kombiniert werden.

Das Acylgerman Bis(4-methoxybenzoyl)diethylgermanium wird bereits in kommerziellen Produkten eingesetzt (Ivocerin^{®} - ein Meilenstein in der Composite-Technologie, Ivoclar Vivadent AG, Report 19, 2013).

Cyklische Acylgermaniumverbindungen, die sich als Photoinitiatoren eignen, sind aus EP 1 905 415 Al bekannt.

Acylgermaniumverbindungen, die mehrere Germaniumatome enthalten, werden in EP 2 103 297 Al offenbart.

Trotz der bereits erzielten Verbesserungen besteht nach wie vor Bedarf an einer weiteren Optimierung der Härtung von Dentalwerkstoffen.

Der Erfindung liegt die Aufgabe zugrunde, Dentalwerkstoffe bereitzustellen, die keine Verfärbungen zeigen, die bei Raumtemperatur eine hohe Lagerstabilität aufweisen und säurestabil sind und die nach der Härtung verbesserte mechanische Eigenschaften aufweisen, d.h. insbesondere eine verbesserte Mund- und Abrasionsstabilität und Haltbarkeit. Außerdem sollen Werkstoffe bereit gestellt werden, die gut aushärten und ein leichtes Entfernen von Materialüberschüssen erlauben und die eine geringe Cytotoxizität aufweisen.

Diese Aufgabe wird erfindungsgemäß durch Dentalwerkstoffe gelöst, die als Initiator für die radikalische Polymerisation eine Kombination aus mindestens einem Thioharnstoffderivat und mindestens einer Bisacyldialkylgermanium-Verbindung enthalten. Dual härtende Dentalwerkstoffe enthalten als zusätzliche Initiatorkomponente zusätzlich ein Peroxid und vorzugsweise ein Hydroperoxid.

Erfindungsgemäß bevorzugte Thioharnstoffderivate werden in US 3,991,008 (Spalte 2, Zeile 35 bis Spalte 3, Zeile 14) und in EP 1 754 465 A1 (Absatz [0009]) beschrieben. Besonders bevorzugte Thioharnstoffderivate sind Methyl-, Ethyl-, Allyl-, Butyl-, Hexyl-, Octyl-, Benzyl-, 1,1,3-Trimethyl-, 1,1-Diallyl, 1,3-Diallyl-, 1-(2-Pyridyl)-2-thioharnstoff, Acetyl-, Propanoyl-, Butanoyl-, Pentanoyl-, Hexanoyl-, Heptanoyl-, Octanoyl, Nonanoyl, Decanoyl und Benzoyl-thioharnstoff, wobei Acetyl- und Hexanoylthioharnstoff ganz besonders bevorzugt sind.

Bevorzugte Bisacyldialkylgermanium-Verbindungen werden in EP 1 905 413 A1, EP 1 905 415 A1 und EP 2 103 297 A1 beschrieben, wobei Bisacylgermane gemäß der Formel (II) der EP 1 905 413 A1 besonders bevorzugt sind. Ganz besonders bevorzugte Bisacyldialkygermanium-Verbindungen sind Bisbenzoyldiethylgermanium, Bisbenzoyldimethylgermanium, Bisbenzoyldibutylgermanium, Bis(4-methoxybenzoyl)dimethylgermanium und Bis(4-methoxybenzoyl)diethylgermanium, wobei Bis(4-methoxybenzoyl)diethylgermanium am meisten bevorzugt ist.

Bevorzugte Hydroperoxide sind 1,1,3,3,-Tetramethylbutylhydroperoxid, t-Butylhydroperoxid, Cumolhydroperoxid, Pinanhydroperoxid, p-Menthanhydroperoxid, Diisopropylbenzolhydroperoxid und t-Amylhydroperoxid, wobei Cumolhydroperoxid besonders bevorzugt ist.

Es wurde überraschenderweise gefunden, dass eine Mischung einer Bisacyldialkylgermanium-Verbindung als Photoinitiator mit einem Thioharnstoff-Derivaten eine verbesserte Aushärtung von Dentalwerkstoffen ermöglicht. Die erfindungsgemäßen Werkstoffe zeichnen sich dabei insbesondere durch einen hohen Aushärtungsgrad, hohe Oberflächenhärte und Abrasionsstabilität, gute Materialstabilität in Gegenwart von Speichel und Langlebigkeit aus.

Photopolymerisierbare Dentalwerkstoffe liegen vorzugsweise als einkomponentige Systeme vor, d.h. in Form einer Mischung, die sämtliche Bestandteile des Dentalwerkstoffs enthält. Sie enthalten als Initiator ausschließlich einen Photoinitiator und lassen such durch Bestrahlung mit Licht härten.

Dualhärtende Dentalwerkstoffe enthalten neben dem Photoinitiator zusätzlich ein Peroxid, vorzugsweise ein Hydroperoxid als Oxidationsmittel. Dualhärtende Werkstoffe liegen bevorzugt in Form von zwei getrennten Komponenten vor, da sonst eine vorzeitige Aushärtung erfolgen würde, wobei die erste Komponente das (Hydro)peroxid und die zweite Komponente das Thioharnstoffderivat enthält. Das Thioharnstoffderivat dient als Reduktionsmittels (Beschleuniger). Die Komponenten werden dementsprechend auch als Katalysator- und als Beschleunigerpaste bezeichnet.

Die Härtung der dual härtenden Werkstoffe kann durch das Mischen von Katalysator- und Beschleunigerpaste ausgelöst werden. Die Zusammensetzung wird so eingestellt, dass sie nach dem Mischen der Pasten noch für einige Minuten (sog. Verarbeitungszeit) verarbeitungsfähig bleibt, nach der Verarbeitung aber schnell aushärtet. Die Verarbeitungs- und Härtungszeit lassen sich vor allem durch Art und Konzentration an (Hydro)peroxid, Thioharnstoff-Derivat und ggf. durch die Zugabe weiteren Komponenten wie Übergangsmetallredoxkatalysator und Inhibitor einstellen.

In der Regel verläuft eine durch Redox-Initiatorsysteme ausgelöste Polymerisation langsamer als eine Photopolymerisation. Dementsprechend lassen sich bei dualhärtenden Werkstoffen Überschüsse leicht entfernen, indem die durch Bestrahlung ausgelöste Photopolymerisation erst nach der Überschussentfernung erfolgt.

Die erfindungsgemäßen Dentalwerkstoffe enthalten eine radikalisch polymerisierbare Matrix. Als polymerisierbare Matrix sind radikalisch polymerisierbare Monomere oder Mischungen radikalisch polymerisierbarer Monomere bevorzugt, insbesondere ein oder mehrere (Meth)acrylate, besonders bevorzugt eine Mischung aus mono- und polyfunktionellen Methacrylaten, ganz besonders bevorzugt aus mono- und difunktionellen Methacrylaten. Unter monofunktionellen (Meth)acrylaten werden Verbindungen mit einer, unter polyfunktionellen (Meth)acrylaten Verbindungen mit zwei oder mehr, vorzugsweise 2 bis 4 radikalisch polymerisierbaren Gruppen verstanden.

Bevorzugte mono- oder polyfunktionelle Methacrylate sind Methyl-, Ethyl-, 2-Hydroxyethyl-, Butyl-, Benzyl-, Tetrahydrofurfuryl- oder Isobornyl(meth)acrylat, p-Cumyl-phenoxyethylenglycolmethacrylat (CMP-1E), Bisphenol-A-dimethacrylat, Bis-GMA (ein Additionsprodukt aus Methacrylsäure und Bisphenol-A-diglycidylether), ethoxy- oder propoxyliertes Bisphenol-A-Dimethacrylat, wie z.B. das Bisphenol-A-Dimethacrylat SR-348c mit 3 Ethoxygruppen oder 2,2-Bis[4-(2-methacryloxypropoxy)phenyl]propan, UDMA (ein Additionsprodukt aus 2-Hydroxyethylmethacrylat (HEMA) und 2,2,4-Trimethylhexamethylendiisocyanat), TMX-UDMA (ein Additionsprodukt aus einer Mischung von HEMA und Hydroxypropylmethacrylat (HPMA) mit α,α,α',α'-Tetramethyl-m-xylylendiisocyanat (TMXDI), Di-, Tri- oder Tetraethylenglycoldimethacrylat, Trimethylolpropantrimethacrylat, Pentaerythrittetramethacrylat, sowie Glycerindi- und trimethacrylat, 1,4-Butandioldimethacrylat, 1,10-Decandioldimethacrylat (D₃MA) oder 1,12-Dodecandioldimethacrylat. Besonders geeignet sind Mischungen aus CMP-1E, UDMA, und TMX-UDMA sowie Glycerindi- bzw. Glycerintrimethacrylat und/oder D₃-MA.

Erfindungsgemäß wird vorzugsweise eine Monomermischung verwendet, die mindestens ein schwerflüchtiges Monomethacrylat, mindestens ein hochviskoses poly-, vorzugsweise difunktionelles Methacrylat und mindestens ein niedrigviskoses poly-, vorzugsweise difunktionelles Methacrylat enthält.

Erfindungsgemäß werden unter schwerflüchtigen Monomeren Verbindungen mit einem Siedepunkt > 150 °C bei Normaldruck verstanden. Der Siedepunkt kann z.B. mit einer Destillationsapparatur bestimmt werden. Unter hochviskosen Monomeren werden Stoffe mit einer Viskosität ≥ 5 Pa·s, vorzugsweise 5 bis 10.000 Pa·s und besonders bevorzugt 5 bis 2.000 Pa·s und unter niedrigviskosen Monomeren Stoffe mit einer Viskosität ≤ 300 mPa·s, vorzugsweise 1 bis 300 mPa·s und besonders bevorzugt 30 bis 300 mPa·s verstanden, wobei die Viskosität mit einem Kapillar- (niedrigviskos) oder Rotationsviskosimeter (hochviskos) bei einer Temperatur von 25°C bestimmt wird.

Besonders bevorzugte hochviskosen Dimethacrylate sind TMX-UDMA (ein Additionsprodukt von HEMA und Hydroxypropylmethacrylat (HPMA) mit α,α,α',α'-Tetramethyl-m-xylylendiisocyanat (TMXDI)) und 1,6-Bis-[2-methacryloyloxyethoxycarbonylamino]-2,4,4-trimethylhexan (UDMA). Bevorzugte niedrigviskose Dimethacrylate, die als Verdünnermonomere genutzten werden, sind Bismethacryloyloxymethyltricyclo[5.2.1.]decan (TCDMA), Glycerindimethacrylat (GDMA) und insbesondere Decandiol-1,10-dimethacrylat (D₃MA). Ein besonders bevorzugtes schwerflüchtiges Monomethacrylat ist p-Cumyl-phenoxyethylenglycolmethacrylat (CMP-1E).

Gemäß einer Ausführungsform enthalten die erfindungsgemäßen Dentalwerkstoffe neben den oben genannten Monomeren zusätzlich ein oder mehrere säuregruppenhaltige radikalisch polymerisierbare Monomere (Haftmonomere). Diese verleihen den Werkstoffen selbsthaftende und/oder selbstätzende Eigenschaften.

Bevorzugte säuregruppenhaltige Monomere sind polymerisationsfähige Carbonsäuren, Phosphonsäuren, Phosphorsäureester, und Sulfonsäuren.

Bevorzugte Carbonsäuren sind Maleinsäure, Acrylsäure, Methacrylsäure, 2-(Hydroxymethyl)acrylsäure, 4-(Meth)acryloyloxyethyltrimellitsäure, 10-Methacryloyloxydecylmalonsäure, N-(2-Hydroxy-3-methacryloyloxypropyl)-N-phenylglycin bzw. 4-Vinylbenzoesäure.

Bevorzugte Phosphonsäuremonomere sind Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methyl-pentylphosphonsäure, 2-[4-(Dihydroxyphosphoryl)-2-oxa-butyl]-acrylsäure oder 2-[4-(Dihydroxyphosphoryl)-2-oxa-butyl]-acrylsäureethyl- und -2,4,6-trimethylphenylester.

Bevorzugte acide polymerisationsfähige Phosphorsäureester sind 2-Methacryloyloxypropylmono- oder -dihydrogenphosphat, 2-Methacryloyloxyethylmono- oder -dihydrogenphosphat, 2-Methacryloyloxyethylphenyl-hydrogenphosphat, Dipentaerythritolpentamethacryloyloxyphosphat, 10-Methacryloyloxydecyl-dihydrogenphosphat, Phosphorsäuremono-(1-acryloyl-piperidin-4-yl)-ester, 6-(Methacrylamido)hexyldihydrogenphosphat und 1,3-Bis-(N-acryloyl-N-propyl-amino)-propan-2-yl-dihydrogenphosphat.

Bevorzugte polymerisationsfähige Sulfonsäuren sind Vinylsulfonsäure, 4-Vinylphenylsulfonsäure oder 3-(Methacrylamido)-propylsulfonsäure.

Besonders bevorzugte Haftmonomere sind 4-(Meth)acryloyloxyethyltrimellitsäure, 2-[4-(Dihydroxyphosphoryl)-2-oxa-butyl]-acrylsäureethyl- oder -2,4,6-trimethylphenylester und 10-Methacryloyloxydecyl-dihydrogenphosphat.

Weiterhin enthalten die erfindungsgemäßen Dentalwerkstoffe vorzugsweise auch organische oder besonders bevorzugt anorganische partikuläre Füllstoffe. Bevorzugt sind Füllstoffe auf der Basis von Oxiden, wie SiO₂, ZrO₂ und TiO₂ bzw. Mischoxiden aus SiO₂, ZrO₂, ZnO und/oder TiO₂, nanopartikuläre oder mikrofeine Füllstoffe, wie pyrogene Kieselsäure oder Fällungskieselsäure (gewichtsmittlere Partikelgröße von 10-1.000 nm) sowie Minifüllstoffe, wie Quarz-, Glaskeramik- oder röntgenopake Glaspulver von z.B. Barium- oder Strontiumaluminiumsilikatgläsern (gewichtsmittlere Partikelgröße von 0,2-10 µm). Weitere Füllstoffe sind röntgenopake Füllstoffe, wie Ytterbiumtrifluorid oder nanopartikuläres Tantal(V)-oxid bzw. Bariumsulfat oder Mischoxide von SiO₂ mit Ytterbium(III)-oxid oder Tantal(V)-oxid (gewichtsmittlere Partikelgröße von 10-1.000 nm).

Zur Verbesserung des Verbundes zwischen den Füllstoffpartikeln und der vernetzten Polymerisationsmatrix können SiO₂-basierende Füllstoffe mit methacrylat-funktionalisierten Silanen, wie z.B. 3-Methacryloyloxypropyltrimethoxysilan, oberflächenmodifiziert werden. Zur Oberflächenmodifizierung von nichtsilikatischen Füllstoffen, z.B. von ZrO₂ oder TiO₂, können auch funktionalisierte saure Phosphate, wie z.B. 10-Methacryloyloxydihydrogenphospaphat eingesetzt werden.

Füllstoffhaltige Dentalwerkstoffe eignen sich besonders als dentale Füllungskomposite und Zemente. Besonders bevorzugt sind Werkstoffe, die nur Füllstoffe mit einer maximalen Partikelgröße von weniger als 600 nm enthalten. Diese eignen sich besonders als dentale Zemente.

Gegebenenfalls können die erfindungsgemäß eingesetzten Zusammensetzungen weitere Additive enthalten, vor allem Stabilisatoren, wie z.B. Polymerisationsstabilisatoren, Farbmittel, mikrobiocide Wirkstoffe, fluoridionenabgebende Additive, optische Aufheller, Fluoreszensmittel, Weichmacher, Übergangsmetallredoxkatalysatoren und/oder UV-Absorber.

Als Übergangsmetallredoxkatalysatoren eignen sich besonders Verbindungen von Übergangsmetallen, die mindestens zwei stabile Wertigkeitsstufen haben. Das sind vor allem Verbindungen der Elemente Kupfer, Eisen, Vanadium, Nickel oder Kobalt, wobei Kupferverbindungen besonders bevorzugt sind, und diese Metalle bevorzugt als gut organolösliche Verbindungen, wie z.B. als Acetylacetonat, Naphthenat oder 2-Ethyl-hexanoat eingesetzt werden. Diese Katalysatoren beschleunigen die Redoxreaktion von Oxidations- und Reduktionsmittel und damit die Radikalbildung, d.h. z.B die Redoxreaktion von Hydroperoxid und Thioharnstoffderivat.

Dentalwerkstoffe, die zusätzlich einen solchen Übergangsmetallredoxkatalysator enthalten, sind erfindungsgemäß bevorzugt. Diese Redoxkatalysatoren werden vorzugsweise in einer Menge von 10-100 ppm, besonders bevorzugt 20-80 ppm und ganz besonders bevorzugt 30-80 ppm eingesetzt, bezogen auf die Gesamtmasse des Werkstoffs.

Erfindungsgemäß bevorzugt sind solche Dentalwerkstoffe, die die folgende Zusammensetzung haben:
(a) 2,0 bis 20 Gew.-%, besonders bevorzugt 5 bis 15 Gew.-% eines oder mehrerer Monomethacrylate,
(b) 5,0 bis 60 Gew.-%, besonders bevorzugt 10 bis 40 Gew.-% eines oder mehrerer Dimethacrylate,
(c) 0 bis 15 Gew.-%, besonders bevorzugt 0 bis 10 Gew.-% eines oder mehrerer säuregruppenhaltiger Haftmonomere,
(d) 20 bis 90 Gew.-%, besonders bevorzugt 40 bis 80 Gew.-% Füllstoff(e),
(e) 0,01 bis 4,0 Gew.-%, besonders bevorzugt 0,1 bis 2,0 Gew.-% Thioharnstoffderivat(e),
(f) 0 bis 3,0 Gew.-%, besonders bevorzugt 0,1 bis 2,0 Gew.-% Hydroperoxid(e),
(g) 0,001 bis 1,0 Gew.-%, besonders bevorzugt 0,005 bis 0,5 Gew.-% Bisacyldialkylgermanium-Verbindung(en),
(h) 0,1 bis 5,0 Gew.-% besonders bevorzugt 0,1 bis 2,0 Gew.-% Additiv(e).

Besonders bevorzugt sind Dentalwerkstoffe, die die folgende Zusammensetzung haben:
(a) 2,0 bis 20 Gew.-%, besonders bevorzugt 5 bis 15 Gew.-% eines oder mehrerer schwerflüchtiger Monomethacrylate,
(b1) 5,0 bis 25 Gew.-%, besonders bevorzugt 5 bis 15 Gew.-% eines oder mehrerer hochviskoser Dimethacrylate,
(b2) 5 bis 30 Gew.-%, besonders bevorzugt 10 bis 20 Gew.-% eines oder mehrerer niedrigviskoser Dimethacrylate,
(c) 0 bis 15 Gew.-%, besonders bevorzugt 0 bis 10 Gew.-% eines oder mehrerer säuregruppenhaltiger Haftmonomere,
(d) 20 bis 90 Gew.-%, besonders bevorzugt 40 bis 80 Gew.-% Füllstoff(e),
(e) 0,01 bis 4,0 Gew.-%, besonders bevorzugt 0,1 bis 2,0 Gew.-% Thioharnstoffderivat(e),
(f) 0 bis 3,0 Gew.-%, besonders bevorzugt 0,1 bis 2,0 Gew.-% Hydroperoxid(e),
(g) 0,001 bis 1,0 Gew.-%, besonders bevorzugt 0,005 bis 0,5 Gew.-% Bisacyldialkylgermanium-Verbindung(en),
(h) 0,1 bis 5,0 Gew.-% besonders bevorzugt 0,1 bis 2,0 Gew.-% Additiv(e).

Wie ausgeführt enthalten die Werkstoffe vorzugsweise 10-100 ppm, besonders bevorzugt 20-80 ppm und ganz besonders bevorzugt 30-80 ppm eines Übergangsmetallredoxkatalysators, wobei diese Mengenbereiche sowohl für die bevorzugten als auch für die besonders bevorzugten Zusammensetzungen gelten.

Dualhärtende Werkstoffe enthalten 0,1 bis 3,0 Gew.-%, besonders bevorzugt 0,1 bis 2,0 Gew.-% Peroxid(e), vorzugsweise Hydroperoxid(e). Die erfindungsgemäßen Dentalwerkstoffe können in ein- oder zweikomponentiger Form bereitgestellt werden. Dualhärtende Werkstoffe sind vorzugsweise zweikomponentig, d.h. sie enthalten zwei getrennte Komponenten, die vor der Anwendung miteinander gemischt werden. Die Zusammensetzung der Komponenten wird so gewählt, dass nach dem Mischen Werkstoffe mit der oben definierten Gesamtzusammensetzung erhalten werden.

Ganz besonders bevorzugt sind solche Dentalwerkstoffe, welche aus den genannten Stoffen bestehen. Weiterhin sind solche Werkstoffe bevorzugt, bei denen die einzelnen Stoffe jeweils aus den oben genannten bevorzugten und besonders bevorzugten Stoffen ausgewählt sind. Besonders bevorzugt sind Werkstoffe, die keine Amine wie z.B. Amin-Beschleuniger, enthalten. Ebenso sind Werkstoffe bevorzugt, die kein TEGDMA und vorzugsweise auch kein Bis-GMA enthalten.

Die erfindungsgemäßen Dentalwerkstoffe eignen sich besonders als dentale Zemente, Füllungskomposite und Verblendmaterialien sowie als Materialien zur Herstellung von Inlays, Onlays, Kronen und Brücken. Die Werkstoffe enthalten vorzugsweise nur Füllstoffe mit einer maximalen Partikelgröße von < 600 nm. Sie gestatten die Herstellung von Dentalwerkstoffen mit geringer Oberflächenrauhigkeit und hohem Glanz sowie ausgezeichneter Abrasionsstabilität.

Die Dentalwerkstoffe eignen sich primär zur intraoralen Anwendung durch den Zahnarzt zur Restauration geschädigter Zähne (klinische Materialien). Sie können aber auch extraoral eingesetzt werden, beispielsweise bei der Herstellung oder Reparatur von Dentalrestaurationen (technische Materialien).

Die Erfindung wird im Folgenden anhand von Ausführungsbeispielen näher erläutert.

### Ausführungsbeispiele

### Beispiele 1-8:

### Lichthärtende Komposite auf der Basis einer erfindungsgemäßen Initiatorzusammensetzung

Entsprechend der nachfolgend aufgeführten Tabelle 1 wurden Komposite (alle Angaben in Masse-%) auf der Basis von 44,5% eines silanisierten SiO₂-Mischoxides mit einem Gehalt von 30% ZrO₂), 20 % Ytterbiumfluorid und 35,5 % einer Methacrylatmischung (20 % CMP-1E, 20 % GDMA, 20 % TMX-UDMA, 25 % UDMA und 14,5 % D₃MA sowie 0,5 % BHT als Stabilisator) hergestellt. Als Initiatorsystem waren die in Tabelle 1 angegebenen Komponenten enthalten. Die Komposite sind mittels eines Kneters (Firma Linden) hergestellt worden. Zur Messung der Vickershärte wurden Metallformen (h = 2mm, Ø = 10mm) mit Komposit gefüllt und mit einer PET-Folie abgedeckt. Die Polymerisation erfolgte durch Bestrahlen von oben mit einer Polymerisationslampe (LED Bluephase; Fa. Ivoclar Vivadent AG; 10 s bei 650 mW/cm²). Die Prüfkörper wurden nach der Herstellung im Trockenschrank bei 37°C für 24 h gelagert und dann die belichtete Oberseite der Prüfkörper zuerst mit einem 2.500er, danach mit einem 4.000er Schleifpapier plan angeschliffen und schließlich mit Polierpaste poliert. Die Messung der Vickershärte erfolgte an der polymerisierten Oberseite mit einem Universalhärteprüfgerät (Modell ZHU0.2; Fa. Zwick/Röll). Pro Prüfkörper wurden je 3 Einzelmessungen durchgeführt. Die resultierenden Mittelwerte sind in der Tabelle 1 angegeben.

Die Ergebnisse belegen eine signifikant erhöhte Vickershärte für Komposite, die neben einem Ge-Photoinitiator (Ivocerin^{®}) ein Thioharnstoffderivat enthalten, im Vergleich zu Kompositen, die Amin-Beschleuniger (EMBO oder DABA) enthalten. Dies bedeutet eine hohe Abrasionsstabilität und ermöglicht die Herstellung von dentalen Restaurationen mit hoher Oberflächenglätte, hohem Glanz und verbesserter Mundstabilität.

**Tabelle 1: Initiatorgehalt im Monomer der Komposite und Vickershärte**

| **Bsp.** | **Ivocerin^{®1)} [Gew.-%]** | **ATU²⁾/Cu³⁾ [Gew.-%]** | **Amin [Gew.-%]** | **Vickershärte (MPa)** |
|---|---|---|---|---|
| 1^{*)} | 0,035 | 0 | 0 | 92,64±4,3 |
| 2 | 0,035 | 1,50/65 | 0 | 129,1±7,7 |
| 3^{*)} | 0,050 | 0 | 0 | 206,2±9,7 |
| 4 | 0,050 | 1,50/65 | 0 | 242,8±10,1 |
| 4a^{*)} | 0 | 1,50/65 | 0 | keine Polymerisation |
| 4b^{*)} | 0 | 1,50/0 | 0 | keine Polymerisation |
| 5^{*)} | 0,035 | 0 | 0,5 EMBO⁴⁾ | 94,0±2,1 |
| 5a^{*)} | 0,035 | 0 | 1,5 EMBO⁴⁾ | 98±11,5 |
| 6^{*)} | 0,035 | 0 | 0,5 DABA⁵⁾ | 100,0±5,8 |
| 6a^{*)} | 0,035 | 0 | 1,5 DABA⁵⁾ | 95±0,5 |
| 7^{*)} | 0,050 | 0 | 0,5 EMBO⁴⁾ | 210,3±15,5 |
| 8^{*)} | 0,050 | 0 | 0,5 DABA⁵⁾ | 196,4±8,4 |

| | | | | |
|---|---|---|---|---|
| *) Vergleichsbeispiel ¹⁾ Bis-(4-methoxybenzoyl)diethylgermanium (Ivoclar Vivadent AG) ²⁾ 1-Acetylthioharnstoff ³⁾ Cu-Acetylacetonat, Cu-Gehalt in ppm ⁴⁾ (4-Dimethylamino)benzoesäureethylester ⁵⁾ N,N-Diethyl-3,5-di-tert.-butylanilin | | | | |

## Patentansprüche

1. Radikalisch polymerisierbarer Dentalwerkstoff, der als Initiator für die radikalische Polymerisation eine Kombination aus einem Thioharnstoffderivat mit der Formel 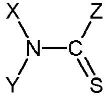 in der
X H oder Y ist,
Y ein Alkylrest mit 1 bis 8 Kohlenstoffatomen, ein Cycloalkylrest mit 5 oder 6 Kohlenstoffatomen, ein Chlor-, Hydroxy- oder Mercapto-substituierter Alkylrest mit 1 bis 8 Kohlenstoffatomen, ein Alkenylrest mit 3 bis 4 Kohlenstoffatomen, ein Arylrest mit 6 bis 8 Kohlenstoffatomen, ein Chlor-, Hydroxy-, Methoxy- oder Sulfonyl-substituierter Phenylrest, ein Acylrest mit 2 bis 8 Kohlenstoffatomen, ein Chlor- oder Methoxy-substituierter Acylrest, ein Aralkylrest mit 7 bis 8 Kohlenstoffatomen oder ein Chlor- oder Methoxysubstituierter Aralkylrest ist, und
Z NH₂, NHX oder NX₂ ist,
einem Übergangsmetallredoxkatalysator und einer Bisacyldialkylgermanium-Verbindung enthält, wobei der Dentalwerkstoff als Bisacyldialkygermanium-Verbindung Bisbenzoyldiethylgermanium, Bisbenzoyldimethylgermanium, Bisbenzoyldibutylgermanium, Bis(4-methoxybenzoyl)dimethylgermanium, Bis(4-methoxybenzoyl)diethylgermanium oder eine Mischung davon enthält.

2. Dentalwerkstoff nach Anspruch 1, der als Thioharnstoffderivat Methyl-, Ethyl-, Allyl-, Butyl-, Hexyl-, Octyl-, Benzyl-, 1,1,3-Trimethyl-, 1,1-Diallyl, 1,3-Diallyl-, 1-(2-Pyridyl-2-thioharnstoff, Acetyl-, Propanoyl-, Butanoyl-, Pentanoyl-, Hexanoyl-, Heptanoyl-, Octanoyl, Nonanoyl, Decanoyl, Benzoyl-thioharnstoff oder eine Mischung davon enthält.

3. Dentalwerkstoff nach einem der vorangehenden Ansprüche, der zusätzlich ein Hydroperoxid enthält.

4. Dentalwerkstoff nach Anspruch 3, der als Hydroperoxid 1,1,3,3,-Tetramethylbutylhydroperoxid, t-Butylperoxid, Cumolhydroperoxid, Pinanhydroperoxid, p-Menthanhydroperoxid, Diisopropylbenzolhydroperoxid, t-Amylhydroperoxid oder eine Mischung davon enthält.

5. Dentalwerkstoff nach einem der Ansprüche 1 bis 4, der als polymerisierbare Matrix radikalisch polymerisierbare Monomere oder Mischungen radikalisch polymerisierbarer Monomere, insbesondere ein oder mehrere Methacrylate, besonders bevorzugt eine Mischung aus mono- und polyfunktionellen Methacrylaten, oder ganz besonders bevorzugt eine Mischung aus mono- und difunktionellen Methacrylaten enthält.

6. Dentalwerkstoff nach Anspruch 5, der eine Monomermischung enthält, die mindestens ein schwerflüchtiges Monomethacrylat, mindestens ein hochviskoses polyfunktionelles, vorzugsweise difunktionelles Methacrylat und mindestens ein niedrigviskoses polyfunktionelles, vorzugsweise difunktionelles Methacrylat enthält.

7. Dentalwerkstoff nach Anspruch 6, der als hochviskoses Dimethacrylat TMX-UDMA (ein Additionsprodukt von HEMA und Hydroxypropylmethacrylat (HPMA) mit α,α,α',α'-Tetramethyl-m-xylylendiisocyanat (TMXDI)) und/oder 1,6-Bis-[2-methacryloyloxyethoxycarbonylamino]-2,4,4-trimethylhexan (UDMA), als niedrigviskoses Dimethacrylat Bismethacryloyloxymethyltricyclo[5.2.1.]decan (TCDMA), Glycerindimethacrylat (GDMA) und/oder Decandiol-1,10-dimethacrylat (D₃MA) und als schwerflüchtiges Monomethacrylat p-Cumyl-phenoxyethylenglycolmethacrylat (CMP-1E) enthält.

8. Dentalwerkstoff nach einem der Ansprüche 1 bis 7, der zusätzlich organischen oder anorganischen partikulären Füllstoff enthält, vorzugsweise mit einer maximalen Partikelgröße von kleiner 600 nm.

9. Dentalwerkstoff nach einem der Ansprüche 1 bis 8, der
(a) 2,0-20 Gew.-%, bevorzugt 5-15 Gew.-% eines oder mehrerer Monomethacrylate,
(b) 5,0-60 Gew.-%, bevorzugt 10-40 Gew.-% eines oder mehrerer Dimethacrylate,
(c) 0-15 Gew.-%, bevorzugt 0-10 Gew.-% eines oder mehrerer säuregruppenhaltiger Haftmonomere,
(d) 20-90 Gew.-%, bevorzugt 40-80 Gew.-% Füllstoff(e),
(e) 0,01-4,0 Gew.-%, bevorzugt 0,1-2,0 Gew.-% Thioharnstoffderivat(e),
(f) 0-3,0 Gew.-%, bevorzugt 0,1-2,0 Gew.-% Hydroperoxid(e),
(g) 0,001-1,0 Gew.-%, bevorzugt 0,005-0,5 Gew.-% Bisacyldialkylgermanium-Verbindung(en),
(h) 0,1-5,0 Gew.-% bevorzugt 0,1-2,0 Gew.-% Additiv(e) enthält.

10. Dentalwerkstoff nach Anspruch 9, der
(a) 2,0-20 Gew.-%, bevorzugt 5-15 Gew.-% eines oder mehrerer schwerflüchtiger Monomethacrylate,
(b1) 5,0-25 Gew.-%, bevorzugt 5-15 Gew.-% eines oder mehrerer hochviskoser Dimethacrylate,
(b2) 5-30 Gew.-%, bevorzugt 10-20 Gew.-% eines oder mehrerer niedrigviskoser Dimethacrylate,
(c) 0-15 Gew.-%, bevorzugt 0-10 Gew.-% eines oder mehrerer säuregruppenhaltiger Haftmonomere,
(d) 20-90 Gew.-%, bevorzugt 40-80 Gew.-% Füllstoff(e),
(e) 0,01-4,0 Gew.-%, bevorzugt 0,1-2,0 Gew.-% Thioharnstoffderivat(e),
(f) 0-3,0 Gew.-%, bevorzugt 0,1-2,0 Gew.-% Hydroperoxid(e),
(g) 0,001-1,0 Gew.-%, bevorzugt 0,005-0,5 Gew.-% Bisacyldialkylgermanium-Verbindung(en),
(h) 0,1-5,0 Gew.-% bevorzugt 0,1-2,0 Gew.-% Additiv(e) enthält.

11. Dentalwerkstoff nach einem der Ansprüche 1 bis 10, der 10-100 ppm, besonders bevorzugt 20-80 ppm und ganz besonders bevorzugt 30-80 ppm Übergangsmetallredoxkatalysator enthält.

12. Dentalwerkstoff nach einem der Ansprüche 1 bis 11, der frei von TEGDMA und vorzugsweise auch von Bis-GMA ist.

13. Dentalwerkstoff nach einem der Ansprüche 1 bis 12, der frei von Aminen ist.

14. Verwendung eines Dentalwerkstoffs gemäß einem der Ansprüche 1 bis 13 als dentaler Zement, Füllungskomposit, Verblendmaterial, als Materialien zur Herstellung von Inlays, Onlays, Kronen oder Brücken.

## Claims

1. Radically polymerizable dental material which comprises as initiator for the radical polymerization a combination of a thiourea derivative having the formula 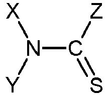 wherein
X is H or Y,
Y is an alkyl radical having 1 to 8 carbon atoms, a cycloalkyl radical having 5 or 6 carbon atoms, a chlorine, hydroxy or mercapto substituted alkyl radical having 1 to 8 carbon atoms, an alkenyl radical having 3 to 4 carbon atoms, an aryl group having 6 to 8 carbon atoms, a chloro, hydroxy, methoxy or sulfonyl substituted phenyl group, an acyl group having 2 to 8 carbon atoms, a chloro or methoxy substituted acyl group, an aralkyl group having 7 to 8 carbon atoms, or a chloro or methoxy substituted aralkyl group, and
Z is NH₂, NHX or NX₂,
a transition metal redox catalysts and a bisacyldialkylgermanium compound, wherein the dental material comprises as bisacyldialkylgermanium compound bisbenzoyldiethylgermanium, bisbenzoyldimethylgermanium, bisbenzoyldibutylgermanium, bis(4-methoxybenzoyl)dimethylgermanium, bis(4-methoxybenzoyl)diethylgermanium or a mixture thereof.

2. Dental material according to claim 1, which comprises as thiourea derivative methyl, ethyl, allyl, butyl, hexyl, octyl, benzyl, 1,1,3-trimethyl, 1,1-diallyl, 1,3-diallyl, 1-(2-pyridyl)-2-thiourea, acetyl, propanoyl, butanoyl, pentanoyl, hexanoyl, heptanoyl, octanoyl, nonanoyl, decanoyl, benzoyl thiourea or a mixture thereof.

3. Dental material according to one of the previous claims, which additionally comprises a hydroperoxide.

4. Dental material according to claim 3, which comprises as hydroperoxide 1,1,3,3-tetramethylbutyl hydroperoxide, t-butyl peroxide, cumene hydroperoxide, pinane hydroperoxide, p-menthane hydroperoxide, diisopropylbenzene hydroperoxide, t-amyl hydroperoxide or a mixture thereof.

5. Dental material according to one of claims 1 to 4, which comprises as polymerizable matrix radically polymerizable monomers or mixtures of radically polymerizable monomers, in particular one or more methacrylates, particularly preferably a mixture of mono- and polyfunctional methacrylates, or quite particularly preferably a mixture of mono- and difunctional methacrylates.

6. Dental material according to claim 5, which comprises a monomer mixture which comprises at least one low-volatile monomethacrylate, at least one highly viscous polyfunctional, preferably difunctional, methacrylate and at least one low-viscosity polyfunctional, preferably difunctional, methacrylate.

7. Dental material according to claim 6, which comprises as highly viscous dimethacrylate TMX-UDMA (an addition product of HEMA and hydroxypropyl methacrylate (HPMA) with a,a,a',a'-tetramethyl-m-xylylene diisocyanate (TMXDI)) and/or 1,6-bis-[2-methacryloyloxyethoxycarbonylamino]-2,4,4-trimethylhexane (UDMA), as low-viscosity dimethacrylate bismethacryloyloxymethyltricyclo[5.2.1.]decane (TCDMA), glycerol dimethacrylate (GDMA) and/or decanediol-1,10-dimethacrylate (D₃MA) and as low-volatile monomethacrylate p-cumylphenoxyethylene glycol methacrylate (CMP-1E).

8. Dental material according to one of claims 1 to 7, which additionally comprises organic or inorganic particulate filler, preferably with a maximum particle size of less than 600 nm.

9. Dental material according to one of claims 1 to 8, which comprises
(a) 2.0-20 wt.-%, preferably 5-15 wt.-% of one or more monomethacrylates,
(b) 5.0-60 wt.-%, preferably 10-40 wt.-% of one or more dimethacrylates,
(c) 0-15 wt.-%, preferably 0-10 wt.-% of one or more acid-group-containing adhesive monomers,
(d) 20-90 wt.-%, preferably 40-80 wt.-% filler(s),
(e) 0.01-4.0 wt.-%, preferably 0.1-2.0 wt.-% thiourea derivative(s),
(f) 0-3.0 wt.-%, preferably 0.1-2.0 wt.-% hydroperoxide(s),
(g) 0.001-1.0 wt.-%, preferably 0.005-0.5 wt.-% bisacyldialkylgermanium compound(s),
(h) 0.1-5.0 wt.-%, preferably 0.1-2.0 wt.-% additive(s).

10. Dental material according to claim 9, which comprises
(a) 2.0-20 wt.-%, preferably 5-15 wt.-% of one or more low-volatile monomethacrylates,
(b1) 5.0-25 wt.-%, preferably 5-15 wt.-% of one or more highly viscous dimethacrylates,
(b2) 5-30 wt.-%, preferably 10-20 wt.-% of one or more low-viscosity dimethacrylates,
(c) 0-15 wt.-%, preferably 0-10 wt.-% of one or more acid-group-containing adhesive monomers,
(d) 20-90 wt.-%, preferably 40-80 wt.-% filler(s),
(e) 0.01-4.0 wt.-%, preferably 0.1-2.0 wt.-% thiourea derivative(s),
(f) 0-3.0 wt.-%, preferably 0.1-2.0 wt.-% hydroperoxide(s),
(g) 0.001-1.0 wt.-%, preferably 0.005-0.5 wt.-% bisacyldialkylgermanium compound(s),
(h) 0.1-5.0 wt.-%, preferably 0.1-2.0 wt.-% additive(s).

11. Dental material according to one of claims 1 to 10, which comprises 10 to 100 ppm, particularly preferably 20 to 80 ppm and most preferably 30 to 80 ppm transition metal redox catalyst.

12. Dental material according to one of claims 1 to 11, which is free from TEGDMA and preferably also from bis-GMA.

13. Dental material according to one of claims 1 to 12, which is free from amines.

14. Use of a dental material according to one of claims 1 to 13 as dental cement, filling composite, veneering material, as materials for preparing inlays, onlays, crowns or bridges.

## Revendications

1. Matériau dentaire polymérisable par voie radicalaire, qui contient, en tant qu'amorceur de polymérisation par voie radicalaire, une combinaison d'un dérivé de thiourée de formule 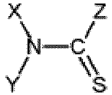 dans laquelle
- X représente un atome d'hydrogène ou une entité symbolisée par Y,
- Y représente un groupe alkyle comportant de 1 à 8 atomes de carbone, un groupe cycloalkyle comportant 5 ou 6 atomes de carbone, un groupe alkyle comportant de 1 à 8 atomes de carbone et porteur d'un ou de substituant(s) chloro, hydroxy ou mercapto, un groupe alcényle comportant de 3 à 4 atomes de carbone, un groupe aryle comportant de 6 à 8 atomes de carbone, un groupe phényle porteur d'un ou de substituant(s) chloro, hydroxy, méthoxy ou sulfonyle, un groupe acyle comportant de 2 à 8 atomes de carbone, un groupe acyle porteur d'un ou de substituant(s) chloro ou méthoxy, un groupe aralkyle comportant de 7 à 8 atomes de carbone ou un groupe aralkyle porteur d'un ou de substituant(s) chloro ou méthoxy,
- et Z représente une entité symbolisée par NH₂, NHX ou NX₂,
d'un catalyseur redox à métal de transition et d'un composé de type bisacyl-dialkyl-germanium, étant entendu que ledit matériau dentaire contient, en tant que composé de type bisacyl-dialkyl-germanium, du bisbenzoyl-diéthyl-germanium, du bisbenzoyl-diméthyl-germanium, du bisbenzoyl-dibutyl-germanium, du bis(4-méthoxy-benzoyl)-diméthyl-germanium, du bis(4-méthoxy-benzoyl)-diéthyl-germanium, ou un mélange de ces composés.

2. Matériau dentaire conforme à la revendication 1, qui contient, en tant que dérivé de thiourée, de la méthyl-thiourée, de l'éthyl-thiourée, de l'allyl-thiourée, de la butyl-thiourée, de l'hexyl-thiourée, de l'octyl-thiourée, de la benzyl-thiourée, de la 1,1,3-triméthyl-thiourée, de la 1,1-diallyl-thiourée, de la 1,3-diallyl-thiourée, de la 1-(pyridin-2-yl)-2-thiourée, de l'acétyl-thiourée, de la propanoyl-thiourée, de la butanoyl-thiourée, de la pentanoyl-thiourée, de l'hexanoyl-thiourée, de l'heptanoyl-thiourée, de l'octanoyl-thiourée, de la nonanoyl-thiourée, de la décanoyl-thiourée, de la benzoyl-thiourée, ou un mélange de ces composés.

3. Matériau dentaire conforme à l'une des revendications précédentes, qui contient en outre un hydroperoxyde.

4. Matériau dentaire conforme à la revendication 3, qui contient, en tant qu'hydroperoxyde, du 1,1,3,3-tétraméthyl-butyl-hydroperoxyde, du tertiobutyl-peroxyde, de l'hydroperoxyde de cumène, de l'hydroperoxyde de pinane, de l'hydroperoxyde de para-menthane, de l'hydroperoxyde de diisopropyl-benzène, du tertioamyl-hydroperoxyde, ou un mélange de ces composés.

5. Matériau dentaire conforme à l'une des revendications 1 à 4, qui contient, en tant que matrice polymérisable, des monomères polymérisables par voie radicalaire ou des mélanges de monomères polymérisables par voie radicalaire, en particulier un ou plusieurs méthacrylate(s), de préférence un mélange de méthacrylates monofonctionnels et polyfonctionnels, et surtout, un mélange de méthacrylates monofonctionnels et difonctionnels.

6. Matériau dentaire conforme à la revendication 5, qui contient un mélange de monomères comprenant au moins un monométhacrylate peu volatil, au moins un méthacrylate polyfonctionnel, de préférence difonctionnel, fortement visqueux, et au moins un méthacrylate polyfonctionnel, de préférence difonctionnel, faiblement visqueux.

7. Matériau dentaire conforme à la revendication 6, qui contient, en tant que diméthacrylate fortement visqueux, du TMX-UDMA (un produit d'addition de HEMA et de HPMA (méthacrylate d'hydroxypropyle) avec du TMXDI (α,α,α',α'-tétraméthyl-méta-xylylène-diisocyanate)) et/ou de l'UDMA (1,6-bis[(2-méthacryloyloxy-éthoxy)-carbonylamino]-2,4,4-triméthyl-hexane ; en tant que diméthacrylate faiblement visqueux, du TCDMA (bis(méthacryloyloxy-méthyl)-tricyclo[5.2.1]décane), du GDMA (diméthacrylate de glycérol) et/ou du D₃MA (diméthacrylate de décane-1,10-diol) ; et en tant que monométhacrylate peu volatil, du CMP-1E (méthacrylate de para-cumyl-phénoxy-éthylène-glycol).

8. Matériau dentaire conforme à l'une des revendications 1 à 7, qui contient en outre une charge organique ou inorganique en particules dont la taille maximale vaut de préférence moins de 600 nm.

9. Matériau dentaire conforme à l'une des revendications 1 à 8, qui contient
a) de 2,0 à 20 % en poids et de préférence de 5 à 15 % en poids d'un ou de plusieurs monométhacrylate(s),
b) de 5,0 à 60 % en poids et de préférence de 10 à 40 % en poids d'un ou de plusieurs diméthacrylate(s),
c) de 0 à 15 % en poids et de préférence de 0 à 10 % en poids d'un ou de plusieurs monomère(s) adhésif(s) porteur(s) de groupes acides,
d) de 20 à 90 % en poids et de préférence de 40 à 80 % en poids de charge(s),
e) de 0,01 à 4,0 % en poids et de préférence de 0,1 à 2,0 % en poids de dérivé(s) de thiourée,
f) de 0 à 3,0 % en poids et de préférence de 0,1 à 2,0 % en poids d'hydroperoxyde(s),
g) de 0,001 à 1,0 % en poids et de préférence de 0,005 à 0,5 % en poids de composé(s) de type bisacyl-dialkyl-germanium,
h) de 0,1 à 5,0 % en poids et de préférence de 0,1 à 2,0 % en poids d'adjuvant(s).

10. Matériau dentaire conforme à la revendication 9, qui contient
a) de 2,0 à 20 % en poids et de préférence de 5 à 15 % en poids d'un ou de plusieurs monométhacrylate(s) peu volatil(s),
b1) de 5,0 à 25 % en poids et de préférence de 5 à 15 % en poids d'un ou de plusieurs diméthacrylate(s) fortement visqueux,
b2) de 5 à 30 % en poids et de préférence de 10 à 20 % en poids d'un ou de plusieurs diméthacrylate(s) faiblement visqueux,
c) de 0 à 15 % en poids et de préférence de 0 à 10 % en poids d'un ou de plusieurs monomère(s) adhésif(s) porteur(s) de groupes acides,
d) de 20 à 90 % en poids et de préférence de 40 à 80 % en poids de charge(s),
e) de 0,01 à 4,0 % en poids et de préférence de 0,1 à 2,0 % en poids de dérivé(s) de thiourée,
f) de 0 à 3,0 % en poids et de préférence de 0,1 à 2,0 % en poids d'hydroperoxyde(s),
g) de 0,001 à 1,0 % en poids et de préférence de 0,005 à 0,5 % en poids de composé(s) de type bisacyl-dialkyl-germanium,
h) de 0,1 à 5,0 % en poids et de préférence de 0,1 à 2,0 % en poids d'adjuvant(s).

11. Matériau dentaire conforme à l'une des revendications 1 à 10, qui contient de 10 à 100 ppm, de préférence de 20 à 80 ppm et surtout de 30 à 80 ppm de catalyseur redox à métal de transition.

12. Matériau dentaire conforme à l'une des revendications 1 à 11, qui ne contient pas de TEGDMA, et de préférence, pas non plus de bis-GMA.

13. Matériau dentaire conforme à l'une des revendications 1 à 12, qui ne contient pas d'amines.

14. Utilisation d'un matériau dentaire conforme à l'une des revendications 1 à 13 en tant que ciment dentaire, composite de remplissage, matériau de placage, en tant que matériaux pour la fabrication de prothèses inlays ou onlays, de couronnes ou de bridges.
